# EUROPEAN PATENT SPECIFICATION

(11) **EP 2 276 423 B1**
(45) Date of publication and mention of the grant of the patent: **20.03.2013**
(21) Application number: 09732613.6
(22) Date of filing: 15.04.2009
(51) Int. Cl.: A61F 2/30, A61F 2/36

(54) **FIBROUS IMPLANTS FOR CARTILAGE REPAIR OR REPLACEMENT**
FASERIGE IMPLANTATE ZUR REPARATUR ODER ZUM ERSATZ VON KNORPEL
IMPLANTS FIBREUX POUR RÉPARATION OU REMPLACEMENT D'UN CARTILAGE

(30) Priority: 15.04.2008 US 45023 P
(43) Date of publication of application: 26.01.2011
(73) Proprietor: Zimmer, Inc., Warsaw, IN 46581-0708 (US)
(72) Inventor: LOWER, Jerry, Bourbon IN 46504 (US); THOMAS, Brian, H., Columbia City IN 46725 (US); MASON, James, Granger IN 46530 (US); MIMNAUGH, Kimberly, North Webster IN 46555 (US)
(74) Representative: Mays, Julie
(86) International application number: PCT/US2009/040621
(87) International publication number: WO 2009/129285

(56) References cited:
- EP-A- 1 491 164
- WO-A-2006/026981
- WO-A-2006/060555

## Description

### BACKGROUND OF THE INVENTION

### 1. Field of the Invention.

The present invention relates to cartilage repair or replacement, and, more particularly, to an orthopedic device for repairing a cartilage defect and a method for utilizing the same.

### 2. Description of the Related Art.

Skeletal joints include articular cartilage to reduce friction, to impart flexibility, and to cushion the impact between opposing articular surfaces. Articular cartilage that becomes damaged may be unable to perform these functions, thereby resulting in pain and restricted motion of the joint. Cartilage may become damaged due to aging, traumatic injury, degenerative disease, such as osteoarthritis, or inflammatory disease, such as rheumatoid arthritis. Once damaged, articular cartilage has a limited ability to heal on its own because it lacks a blood supply. Therefore, devices and methods for repairing cartilage defects are extremely important for relieving pain and restoring motion of the joint.

Various techniques have been developed to relieve pain and restore motion in skeletal joints. Typically, the ends of the bones forming a joint are cut away and replaced with prosthetic implants. Just like the bone it replaced, the implant itself must have a surface that is capable of interacting with adjacent anatomical structures, such as surrounding cartilage and opposing articular surfaces.

In other cases, when the cartilage defect is small, a patch of replacement cartilage is attached to the bone. This second technique presents its own challenges. For one, it is difficult to attach replacement cartilage to the bone while retaining a surface that is capable of interacting with adjacent anatomical structures. For example, a smooth, elastic polymer may be screwed into the bone, but the head of the screw is now exposed to adjacent anatomical structures. Also, viable cartilage is often sacrificed. In processes such as mosaicplasty and osteochondral autograft transfer, viable cartilage is taken from one area of the body and implanted at the site of the defect. In other cases, viable cartilage must be removed from the site of the defect to produce an evenly-shaped, standard-sized defect to accommodate a standard-sized device.

EP1491164 A1 discloses a prosthetic device for repairing or replacing cartilage or cartilage-like tissue comprising at least one layer of highly-oriented fibres, a base component and a stabilisation area provided in between.

### SUMMARY

According to present invention, the orthopedic device includes an implant securable to a bone. The implant has an exterior substrate. The orthopedic device further includes a bearing portion that extends from the exterior substrate of the implant. The bearing portion has a bearing surface that is capable of articulating against adjacent anatomical structures. This bearing surface includes a plurality of fibers. The bearing portion is integrally molded with the implant.

The design of the present orthopedic device allows for attachment of a bearing portion to bone while retaining a surface that is capable of interacting with adjacent anatomical structures. In addition, the design eliminates the need to remove viable cartilage from other areas of the body, and most importantly, the need to remove viable cartilage from areas surrounding the defect. The defect need not be evenly-shaped nor standard-sized. Also, the design of the present orthopedic device allows for implantation into subchondral bone, which may stimulate the production of fibrocartilaginous tissue. In turn, this fibrocartilaginous tissue may interdigitate with the bearing portion of the orthopedic device, which would help to repair the defect and help to secure the orthopedic device in place. The bearing portion may also contain lubricants to promote interaction with fibrocartilaginous tissue and adjacent anatomical structures. Further, the implantation may be performed arthroscopically. Minimally invasive procedures often reduce recovery time and increase the rate of surgical success.

### BRIEF DESCRIPTION OF THE DRAWINGS

The above-mentioned and other features and advantages of this invention, and the manner of attaining them, will become more apparent and the invention itself will be better understood by reference to the following descriptions of embodiments of the invention taken in conjunction with the accompanying drawings, wherein:

Figure 1 is a cross-sectional view of an orthopedic device of the present invention secured to a femur;

Figure 2 is a cross-sectional view of an orthopedic device of the present invention;

Figure 3 is a cross-sectional view similar to Figure 2 of another orthopedic device of the present invention;

Figure 4 is a cross-sectional view similar to Figure 2 of yet another orthopedic device of the present invention;

Figure 5 is a cross-sectional view of another orthopedic device of the present invention secured to a femur, where the device includes an anchor;

Figure 6 is a plan view of the orthopedic device of Figure 5;

Figure 7 is a cross-sectional view of yet another orthopedic device of the present invention secured to a femur, where the device includes an anchor;

Figure 8 is a partial plan view of the orthopedic device of Figure 7;

Figure 9 is a partial plan view similar to Figure 8 of another orthopedic device of the present invention, where the device includes an anchor;

Figure 10 is a partial plan view of another orthopedic device of the present invention, where the device includes an anchor;

Figure 11 is a partial plan view of another orthopedic device of the present invention, where the device includes an anchor;

Figure 12 is a partial plan view of another orthopedic device of the present invention, where the device includes an anchor;

Figure 13 is a partial plan view similar to Figure 12 of another orthopedic device of the present invention, where the device includes an anchor;

Figure 14 is a cross-sectional view of yet another orthopedic device of the present invention secured to a femur, where the device includes an anchor;

Figure 15 is a plan view of an orthopedic device;

Figure 16 is a plan view of an orthopedic device of the present invention;

Figure 17 is a plan view similar to Figure 16 of another orthopedic device of the present invention;

Figure 18 is a partial cross-sectional view of another orthopedic device of the present invention;

Figure 19 is a partial plan view of another orthopedic device of the present invention, where the device includes an anchor;

Figure 20 is a plan view similar to Figure 16 of another orthopedic device of the present invention; and

Figure 21 is a plan view similar to Figure 16 of another orthopedic device of the present invention.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

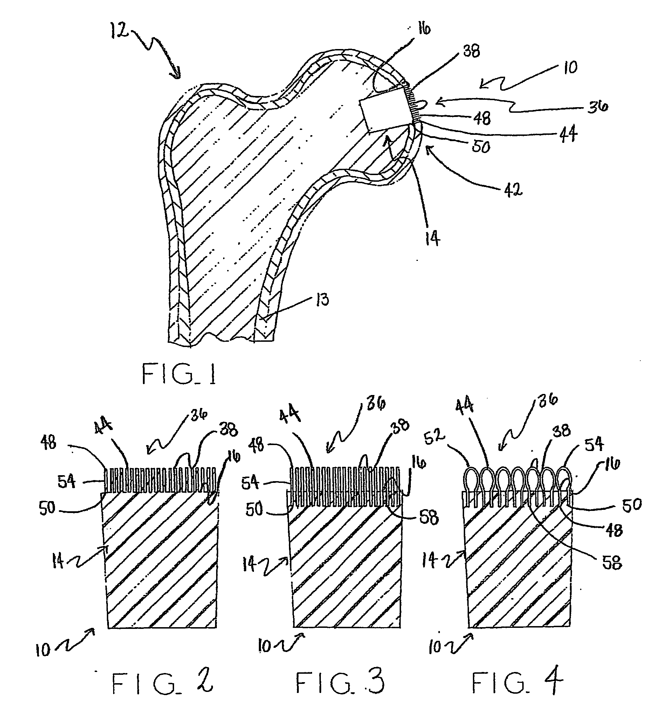

Referring to Figure 1, orthopedic device 10 for repairing a cartilage defect is provided. Orthopedic device 10 may be used to repair both small cartilage defects (smaller in size than approximately 2cm²) and large cartilage defects (equal to or larger in size than approximately 2cm²). As illustrated in Figure 1, orthopedic device 10 is secured to femur 12. While orthopedic device 10 is described and depicted herein as being secured to femur 12, orthopedic device 10 may be secured in other locations and to other types of bones in accordance with the teachings herein. For example, orthopedic device 10 may be secured to the tibia, pelvis, humerus, ulna, radius, tarsus, metatarsus, scapula, clavicle, fibula, talus, vertebral bodies, and phalanges.

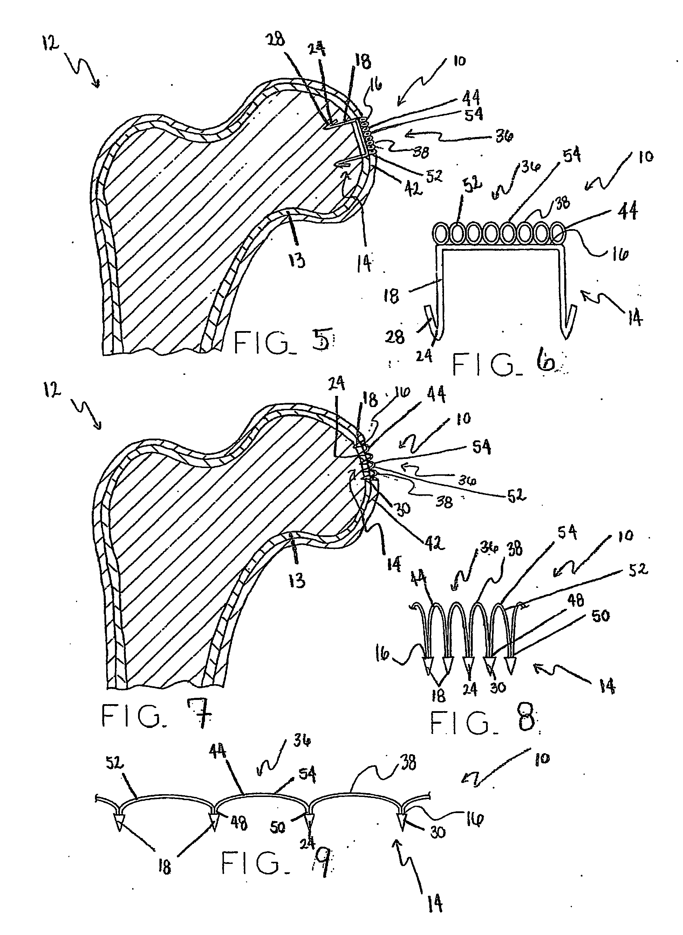

Orthopedic device 10 includes implant 14. Implant 14 includes exterior substrate 16 and is capable of being secured to a bone such as femur 12. Implant 14 may be secured to femur 12 by any method known in the art. In one embodiment of the present invention, illustrated in Figure 1, implant 14 may be secured to femur 12 by press fitting implant 14 to femur 12. Implant 14 may be configured to swell after implantation into femur 12 to enhance fixation. In another embodiment, implant 14 may be secured to femur 12 with an adhesive, such as bone cement. In another embodiment, illustrated in Figures 5, 7, and 14, implant 14 may be secured to femur 12 with anchor 18. Anchor 18 is described in more detail below. In yet another embodiment, implant 14 may be secured to an adjacent orthopedic device (not shown) already implanted in femur 12 by a mechanical connection. For example, implant 14 may take the form of a prosthetic femoral head capable of being secured to femur 12 by a mechanical connection to a femoral hip stem that is already implanted into femur 12.

According to an exemplary embodiment of the present invention, the attachment between implant 14 and femur 12 may be enhanced by ingrowth of femur 12 bone tissue into a porous implant 14. This exemplary embodiment is described in more detail below.

According to another exemplary embodiment of the present invention, implant 14 may be implanted into subchondral layer 13 of femur 12, which may in turn stimulate the production of fibrocartilaginous tissue, the benefits of which are discussed in more detail below. In the accompanying drawings, the scale of orthopedic device 10 may be exaggerated relative to the scale of femur 12. For example, Figure 1 shows implant 14 extending well-beyond subchondral layer 13 of femur 12 when, in use, it may be positioned within subchondral layer 13 of femur 12.

According to yet another exemplary embodiment of the present invention, implant 14 may be secured to femur 12 using arthroscopic surgical procedures. Such procedures often reduce a patient's recovery time and increase the rate of surgical success. Implant 14 may be cannulated to permit arthroscopic insertion.

As shown in Figures 5-14, implant 14 may include anchor 18. Anchor 18 may be any component capable of attachment to bone. In other words, anchor 18 should be capable of being driven into bone, and once driven inside, capable of remaining in the bone. For example, bone-attaching end 24 of anchor 18 may take the shape of screw 26, barb 28, spike 30, sphere 32, or partial sphere 34. As with the attachment between implant 14 and femur 12, the attachment between anchor 18 and femur 12 may be enhanced by ingrowth of femur 12 bone tissue into a porous anchor 18, as described in more detail below.

Implant 14 may be constructed of any biocompatible material. Implant 14 may be constructed of a resilient material, such as a polymer, specifically, a hydrogel or another hydrophilic polymer, or implant 14 may be constructed of a rigid material, such as ceramic or metal. It is also within the scope of the present invention that implant 14 may be constructed of a variety of materials. For example, implant 14 may transition from a rigid material near substrate 16 to a resilient material away from substrate 16 to reduce the likelihood that implant 14 will become loosened from femur 12. The subchondral surface or end of the implant (i.e., the portion of the implant oriented toward cancellous bone) may be softer than the chondral surface or end of the implant (i.e., the portion of the implant oriented toward the cartilage and/or the surface of the bone) so that the implant may lock into the cancellous bone and not be readily removed or pulled out by suction or similar force. Similarly, anchor 18 of implant 14 may be constructed of any biocompatible material. In an exemplary embodiment, because anchor 18 is designed for attachment to femur 12, anchor 18 will be constructed of a rigid material, such as ceramic or metal. Implant 14 and/or anchor 18 may be configured to swell upon implantation into femur 12 to enhance fixation to femur 12.

Metals that may be used to form implant 14 and anchor 18 include, but are not limited to, titanium, a titanium alloy, cobalt chromium, cobalt chromium molybdenum, porous tantalum, or a highly porous biomaterial. A highly porous biomaterial is useful as a bone substitute and as cell and tissue receptive material. As mentioned above, constructing implant 14 and anchor 18 of a highly porous biomaterial may enhance attachment of implant 14 and anchor 18 with femur 12 by the ingrowth of femur 12 bone tissue into implant 14 and anchor 18.

A highly porous biomaterial may have a porosity as low as 55, 65, or 75 percent and as high as 80, 85, or 90 percent, by volume. An example of such a material is produced using Trabecular Metal™ technology generally available from Zimmer, Inc., of Warsaw, Indiana. Trabecular Metal™ is a trademark of Zimmer Technology, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861. In addition to tantalum, other metals such as niobium, or alloys of tantalum and niobium with one another or with other metals may also be used.

Generally, the porous tantalum structure includes a large plurality of ligaments defining open spaces therebetween, with each ligament generally including a carbon core covered by a thin film of metal such as tantalum, for example. The open spaces between the ligaments form a matrix of continuous channels having no dead ends, such that growth of cancellous bone through the porous tantalum structure is uninhibited. The porous tantalum may include up to 75%-85% or more void space therein. Thus, porous tantalum is a lightweight, strong porous structure which is substantially uniform and consistent in composition, and closely resembles the structure of natural cancellous bone, thereby providing a matrix into which cancellous bone may grow to provide fixation of implant 14 and anchor 18 to femur 12.

The porous tantalum structure may be made in a variety of densities in order to selectively tailor the structure for particular applications. In particular, as discussed in the above-mentioned U.S. Patent No. 5,282,861, the porous tantalum may be fabricated to virtually any desired porosity and pore size, and can thus be matched with the surrounding natural bone in order to provide an improved matrix for bone ingrowth and mineralization.

Referring again to Figure 1, orthopedic device 10 further includes bearing portion 36 that extends from substrate 16 of implant 14. Bearing portion 36 includes bearing surface 38 that articulates against adjacent anatomical structures, such as surrounding cartilage 42 and opposing articular surfaces (not shown). For example, when implant 14 of orthopedic device 10 is secured to the head of femur 12, as illustrated in Figure 1, bearing portion 36, and more specifically bearing surface 38 of bearing portion 36, would articulate against the acetabulum of the pelvis. In an exemplary embodiment, bearing portion 36 will interdigitate with fibrocartilaginous tissue, the production of which may be stimulated by implanting a porous implant 14 of orthopedic device 10 into subchondral layer 13 of femur 12, as mentioned above. The interaction between fibrocartilaginous tissue and bearing portion 36 could help to repair a cartilage defect and could help to secure orthopedic device 10 in place.

Bearing portion 36 may be constructed of any biocompatible material. In an exemplary embodiment of the present invention, bearing portion 36, or a portion thereof, may be constructed of a resilient polymer material, such as hydrogel, poly ether ether ketone, fiber reinforced poly ether ether ketone, ultrahigh molecular weight polyethylene, crosslinked ultrahigh molecular weight polyethylene, polyether ketone ether ether ketone, polyaryl ether ketone, polyphenylene, polyacrylate, polymethacrylate, or polyurethane, or combinations or modifications therefrom. Generally, a hydrogel is a network of polymer chains that are watersoluble but made insoluble through physical and/or chemical crosslinks. These materials are sometimes found as a colloidal gel in which water is the dispersion medium. Hydrogels are generally formed from natural or synthetic polymers. Hydrogels may be classified as "superabsorbent" and may contain over 99% water, by weight. In addition, hydrogels may have the abilty to swell due to water absorption. Hydrogels may also possess a degree of flexibility very similar to natural tissue, due to their significant water content. Advantageously, utilizing a resilient material for bearing portion 36 reduces wear on adjacent anatomical structures, such as surrounding cartilage 42 and opposing articular surfaces. Bearing portion 36 may also include lubricants to promote interaction with these adjacent anatomical structures.

Referring still to Figure 1, bearing portion 36 includes fibers 44. More specifically, bearing surface 38 of bearing portion 36 includes fibers 44. Fibers 44 may extend from bearing portion 36 to articulate against adjacent anatomical structures, such as surrounding cartilage 42 and opposing articular surfaces. As used herein, "fiber" means any elongate, thread-like structure. Fibers 44 may come in any size, ranging in diameter 46 or length 47 from mere nanometers to several centimeters. As used herein, "diameter" of fiber 44 is essentially the thickness of fiber 44, as fiber 44 need not have a circular cross-section. As used herein, "length" of fiber 44 is the distance between first end 48 and second end 50 of fiber 44. Synthetic fibers 44 may be formed by melt spinning, dry spinning, wet spinning, or electrospinning. Fibers 44 of the present invention may range between approximately 1 denier and 3000 deniers, and more specifically between approximately 200 deniers and 1700 deniers. Fibers 44 may be absorbable by mammalian, preferably human, tissue, e.g., bone or cartilage.

In an exemplary embodiment, bearing portion 36, including fibers 44, is formed from one or more hydrogels and is substantially free of other polymer materials. In another exemplary embodiment, bearing portion 36 including fibers 44, comprises at least about 25% by weight of one or more hydrogels. In still another exemplary embodiment, bearing portion 36 including fibers 44, comprises at least about 50% by weight of one or more hydrogels. And in still another exemplary embodiment, bearing portion 36 including fibers 44, comprises at least about 75% by weight of one or more hydrogels. In still another exemplary embodiment, bearing portion 36 including fibers 44, comprises at least about 95% by weight of one or more hydrogels.

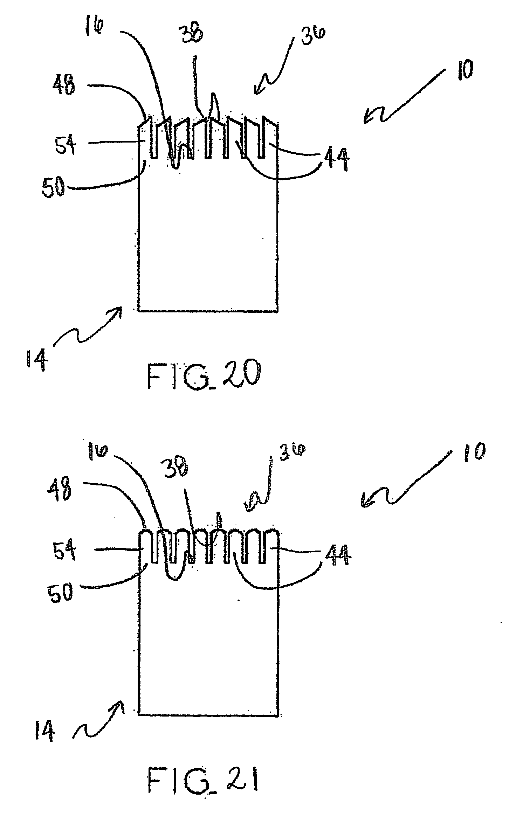

According to an embodiment of the present invention, illustrated in Figures 1-3, first end 48 of fiber 44 articulates against adjacent anatomical structures, while second end 50 of fiber 44 is secured to implant 14. Second end 50 of fiber 44 may be secured to a non-fibrous section of bearing portion 36 which is in turn attached to substrate 16 of implant 14, or second end 50 of fiber 44 may be secured directly to implant 14, as shown in Figures 2-3. First end 48 may include features to enhance interaction with adjacent anatomical structures, such as beveling, as shown in Figure 20, or rounded edges, as shown in Figure 21. Fiber 44 may extend linearly between first end 48 and second end 50, or fiber 44 may be crimped between first end 48 and second end 50. The crimped design may aid fibers 44 in absorbing an impact, permitting fibers 44 to interlock. After implantation of the devices shown in Figures 1-3, first end 48 of fiber 44 remains free, i.e., it remains unconnected to the adjacent anatomical structures with which it articulates. Thus, with reference to Figure 1, fiber 44 does not fasten device 10 or the bone to which it is secured (femur 12) to any other anatomical structure.

According to another embodiment of the present invention, illustrated in Figures 4-14, both first end 48 and second end 50 of fiber 44 are secured to implant 14, thereby forming looped fiber 52. In this form, middle portion 54 of fiber 44, located between first end 48 and second end 50, articulates against adjacent anatomical structures. It is within the scope of the present invention that first end 48 and/or second end 50 of fiber 44 may be secured to a non-fibrous section of bearing portion 36 which is in turn attached to substrate 16 of implant 14, or first end 48 and/or second end 50 may be secured directly to implant 14 as shown in Figure 4. After implantation of the devices shown in Figures 1-14, looped fiber 52 remains free, i.e., it remains unconnected to the adjacent anatomical structures with which it articulates. Thus, with reference to Figure 5 for example, looped fiber 52 does not fasten device 10 or the bone to which it is secured (femur 12) to any other anatomical structure.

According to yet another embodiment of the present invention, fibers 44 of bearing portion 36 may be secured to implant 14 in a variety of ways. For example, first ends 48 of some fibers 44 may be positioned to articulate against adjacent anatomical structures, while middle portion 54 of other fibers 44 may be positioned to articulate against adjacent anatomical structures. In other words, some, but not all, fibers 44 of bearing portion 36 may take the form of looped fibers 52.

In examples not forming part of the invention, bearing portion 36 may be secured to implant 14 by any method known in the art, such as by press fitting, by injection molding, or by adhering bearing portion 36 to implant 14. Fibers 44 of bearing portion 36 may also aid in the attachment between bearing portion 36 and implant 14. For example, fibers 44 of bearing portion 36 may be press fit or injection molded into substrate 16 of implant 14, as shown in Figure 3. In an exemple embodiment, implant 14 includes elongate apertures 58 extending there through. Elongate apertures 58 are capable of receiving either first end 48, or both first end 48 and second end 50, of fibers 44. Therefore, fibers 44 of bearing portion 36 may be press fit or injection molded into elongate apertures 58 in implant 14.

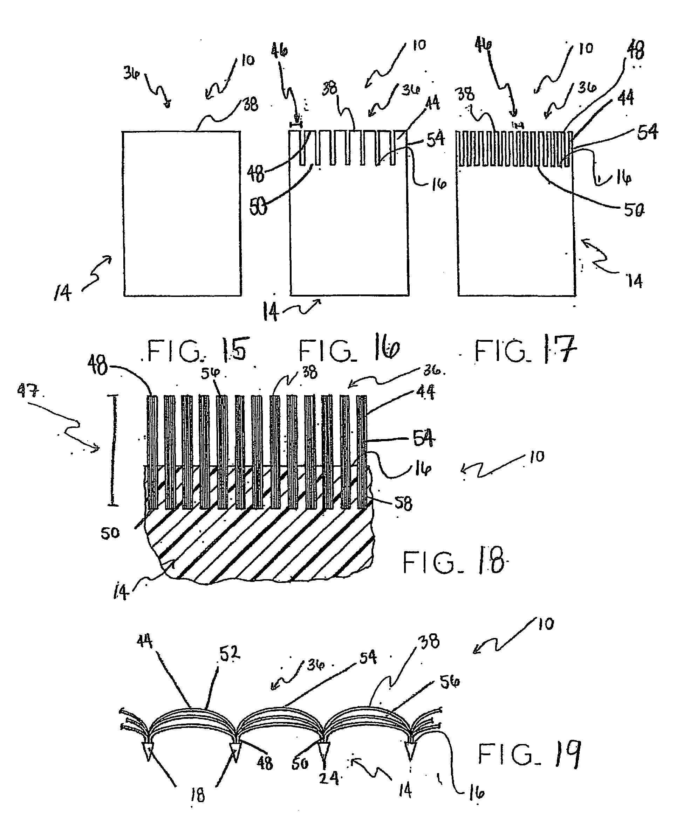

According to the present invention, bearing portion 36 is secured to implant 14 by integrally molding bearing portion 36 with implant 14. Fibers 44 of an integrally molded orthopedic device 10 may be formed in various ways. For example, a single mold may form the shape of both implant 14 and bearing portion 36, including fibers 44. The end result of such a mold would be, for instance, orthopedic device 10 illustrated in Figure 2. As another example, a single mold may form both implant 14 and bearing portion 36, but not fibers 44. The end result of such a mold would be, for instance, orthopedic device 10 illustrated in Figure 15. Then, fibers 44 may be formed by scoring bearing surface 38 of orthopedic device 10, resulting in, for instance, orthopedic device 10 illustrated in Figures 16 and 17.

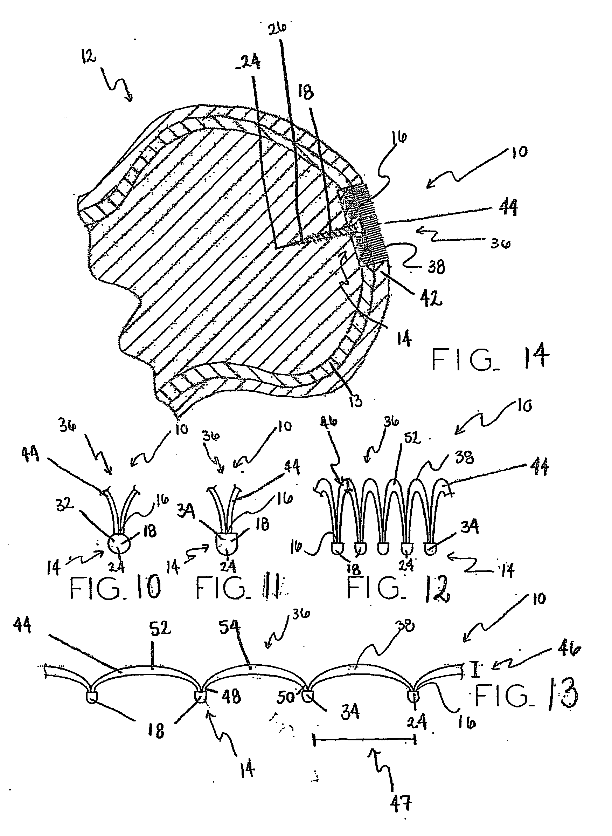

Advantageously, orthopedic device 10 may be designed to accommodate the needs of a particular patient, which could be determined by the size, location, and cause of his or her cartilage defect. In general, the dimensions and density of fibers 44 may be designed to accommodate those needs. The following examples are merely illustrative, as orthopedic device 10, and specifically fibers 44, may be varied in other ways in accordance with the teachings herein. First, diameter 46 of fibers 44 may be varied, as shown by comparing Figures 16 and 17. Thicker fibers 44, like those shown in Figure 16, may provide more resistance than thinner fibers 44, like those shown in Figure 17. Diameter 46 of a single fiber 44 may even vary across its length 47, as shown in Figures 12-13. Second, length 47 of fibers 44 may vary. It may be necessary to have longer fibers 44, and in turn a thicker bearing portion 36, in larger joints, while it may be necessary to have shorter fibers 44, and in turn a thinner bearing portion 36, in more compact joints. Third, individual fibers 44 may be spaced apart across bearing portion 36, as shown in Figures 2 and 8, or multiple fibers 44 may be spaced together in bundles 56 or clusters, as shown in Figures 18-19. It may be necessary to have a more dense arrangement of fibers 44, like those shown in Figures 18-19, in a high-impact joint, while it may be possible to have a less dense arrangement of fibers 44, like those shown in Figures 2 and 8, in other areas.

Also advantageously, without even having to modify its actual design, orthopedic device 10 may accommodate the needs of a particular patient simply by varying the method by which orthopedic device 10 is secured to femur 12. For example, as shown by comparing Figures 8 and 9, the density of fibers 44 can be customized by varying the spacing between adjacent anchors 18. Orthopedic devices 10 of Figures 8-9 may be identical in form, but the density of fibers 44 may be customized simply by changing the distance between adjacent anchors 18. The ability to space implants 14, or anchors 18 of implants 14, in any possible configuration leads to the following advantages. First, the defect need not be evenly-shaped nor standard-sized. Implants 14 may be scattered around even an odd-shaped defect. Second, a single orthopedic device 10 may be adapted to produce a more dense arrangement or a less dense arrangement of fibers 44, depending on, for example, the location of the defect. For example, if a surgeon sees a defect and determines that a more dense arrangement of fibers 44 than originally planned is necessary, the surgeon could accomplish a more dense arrangement of fibers 44, without having to implant a different device.

While this invention has been described as having preferred designs, the present invention can be further modified within the scope of the appended claims.

## Claims

1. An orthopedic device (10) for repairing a cartilage defect, comprising:
an implant (14) securable to a bone, the implant (14) comprising an exterior substrate (16); and
a bearing portion (36) extending from the exterior substrate (16) of the implant (14), the bearing portion (36) comprising a bearing surface (38) configured to articulate against adjacent anatomical structures, said bearing surface (38) comprising a plurality of fibers (44) extending from the bearing portion (36) and **characterised in that** the bearing portion (36) is integrally molded with the implant (14).

2. The device of claim 1, wherein the implant (14) further comprises at least one anchor (18).

3. The device of claim 2, wherein the at least one anchor (18) includes an end in the shape of one of a screw, a barb, a spike, a sphere, and a partial sphere.

4. The device of claim 1, wherein at least one of the plurality of fibers (44) is formed by scoring the bearing portion (36).

5. The device of claim 1, wherein at least one of the plurality of fibers (44) is embedded into the implant (14).

6. The device of claim 1, wherein at least one of the plurality of fibers (44) is press fit into the implant (14).

7. The device of claim 1, wherein at least one of the plurality of fibers (44) has a first end and a second end, the first end configured to articulate against adjacent anatomical structures and the second end being secured to the implant (14).

8. The device of claim 7, wherein the first end of the at least one fiber (44) is beveled.

9. The device of claim 1, wherein the implant (14) includes at least one elongate aperture extending therethrough; and the bearing portion (36) is injection molded into the at least one elongate aperture of the implant.

10. The device of claim 1, wherein the bearing portion (36) comprises a hydrogel.

11. The device of claim 1, wherein the bearing portion (36) further comprises a lubricant.

12. The device of claim 1, wherein at least one of the plurality of fibers (44) forms a loop.

13. The device of claim 1, wherein the plurality of fibers (44) comprises at least one bundle of fibers.

14. The device of claim 1, wherein a diameter of at least one of the plurality of fibers varies across a length of said at least one fiber (44).

## Patentansprüche

1. Orthopädische Vorrichtung (10) zur Reparatur eines Knorpeldefekts, mit:
einem Implantat (14), das an einem Knochen befestigbar ist, wobei das Implantat (14) ein äußeres Substrat (16) aufweist; und
einem Lagerabschnitt (36), der sich vom äußeren Substrat (16) des Implantats (14) erstreckt, wobei der Lagerabschnitt (36) eine Lagerfläche (38) aufweist, die konfiguriert ist, gegen benachbarte anatomische Strukturen ein Gelenk zu bilden, wobei die Lagerfläche (38) mehrere Fasern (44) aufweist, die sich vom Lagerabschnitt (36) erstrecken, und **dadurch gekennzeichnet ist, dass** der Lagerabschnitt (36) integral mit dem Implantat (14) ausgeformt ist.

2. Vorrichtung nach Anspruch 1, wobei das Implantat (14) ferner mindestens eine Verankerung (18) aufweist.

3. Vorrichtung nach Anspruch 2, wobei die mindestens eine Verankerung (18) ein Ende in der Form einer Schraube, eines Widerhakens, eines Nagels, einer Kugel und einer Teilkugel aufweist.

4. Vorrichtung nach Anspruch 1, wobei mindestens eine der mehreren Fasern (44) durch Kerbung des Lagerabschnitts (36) ausgebildet ist.

5. Vorrichtung nach Anspruch 1, wobei mindestens eine der mehreren Fasern (44) in das Implantat (14) eingebettet ist.

6. Vorrichtung nach Anspruch 1, wobei mindestens eine der mehreren Fasern (44) in das Implantat (14) eingepresst ist.

7. Vorrichtung nach Anspruch 1, wobei mindestens eine der mehreren Fasern (44) ein erstes Ende und ein zweites Ende aufweist, wobei das erste Ende konfiguriert ist, gegen benachbarte anatomische Strukturen ein Gelenk zu bilden und das zweites Ende am Implantat (14) befestigt ist.

8. Vorrichtung nach Anspruch 7, wobei das erste Ende der mindestens einen Faser (44) abgeschrägt ist.

9. Vorrichtung nach Anspruch 1, wobei das Implantat (14) mindestens eine längliche Öffnung aufweist, die sich dort hindurch erstreckt; und der Lagerabschnitt (36) in die mindestens eine längliche Öffnung des Implantats spritzgegossen ist.

10. Vorrichtung nach Anspruch 1, wobei der Lagerabschnitt (36) ein Hydrogel aufweist.

11. Vorrichtung nach Anspruch 1, wobei der Lagerabschnitt (36) ferner ein Schmiermittel aufweist.

12. Vorrichtung nach Anspruch 1, wobei mindestens eine der mehreren Fasern (44) eine Schlinge bildet.

13. Vorrichtung nach Anspruch 1, wobei die mehreren Fasern (44) mindestens Faserbündel aufweisen.

14. Vorrichtung nach Anspruch 1, wobei ein Durchmesser mindestens einer der mehreren Fasern über eine Länge der mindestens einen Faser (44) variiert.

## Revendications

1. Dispositif orthopédique (10) destiné à réparer un défaut de cartilage, comprenant :
un implant (14) pouvant être fixé sur un os, l'implant (14) comprenant un substrat extérieur (16) ; et
une partie de palier (36) s'étendant à partir du substrat extérieur (16) de l'implant (14), la partie de palier (36) comprenant une surface de palier (38) configurée pour s'articuler contre des structures anatomiques adjacentes, ladite surface de palier (38) comprenant une pluralité de fibres (44) s'étendant à partir de la partie de palier (36) et **caractérisé en ce que** la partie de palier (36) est venue de moulage avec l'implant (14).

2. Dispositif de la revendication 1, dans lequel l'implant (14) comprend en outre au moins une ancre (18).

3. Dispositif de la revendication 2, dans lequel l'au moins une ancre (18) comporte une extrémité sous forme d'un élément parmi une vis, un barbillon, une pointe, une sphère, et une sphère partielle.

4. Dispositif de la revendication 1, dans lequel au moins une fibre parmi la pluralité de fibres (44) est formée en faisant des incisions dans la partie de palier (36).

5. Dispositif de la revendication 1, dans lequel au moins une fibre parmi la pluralité de fibres (44) est incorporée dans l'implant (14).

6. Dispositif de la revendication 1, dans lequel au moins une fibre parmi la pluralité de fibres (44) est insérée par pression dans l'implant (14).

7. Dispositif de la revendication 1, dans lequel au moins une fibre parmi la pluralité de fibres (44) présente une première extrémité et une deuxième extrémité, la première extrémité étant configurée pour s'articuler contre des structures anatomiques adjacentes et la deuxième extrémité étant fixée à l'implant (14).

8. Dispositif de la revendication 7, dans lequel la première extrémité de l'au moins une fibre (44) est biseautée.

9. Dispositif de la revendication 1, dans lequel l'implant (14) comporte au moins une ouverture allongée s'étendant à travers celui-ci ; et la partie de palier (36) est moulée par injection dans l'au moins une ouverture allongée de l'implant.

10. Dispositif de la revendication 1, dans lequel la partie de palier (36) comprend un hydrogel.

11. Dispositif de la revendication 1, dans lequel la partie de palier (36) comprend en outre un lubrifiant.

12. Dispositif de la revendication 1, dans lequel au moins une fibre parmi la pluralité de fibres (44) forme une boucle.

13. Dispositif de la revendication 1, dans lequel la pluralité de fibres (44) comprend au moins un faisceau de fibres.

14. Dispositif de la revendication 1, dans lequel un diamètre d'au moins une fibre parmi la pluralité de fibres varie le long d'une longueur de ladite au moins une fibre (44).
